# EUROPÄISCHE PATENTSCHRIFT

(11) **EP 1 303 631 B1**
(45) Veröffentlichungstag und Bekanntmachung des Hinweises auf die Patenterteilung: **26.05.2010**
(21) Anmeldenummer: 01951629.3
(22) Anmeldetag: 23.06.2001
(51) Int. Cl.: C12P 11/00, C12P 13/00, C12P 17/12, C12P 17/14, C12P 35/00, C12P 37/00, C07C 235/60, C07D 501/18, C07D 499/21, C07D 499/68, C12N 9/02

(54) **BIOTRANSFORMATION VON BIOLOGISCH AKTIVEN VERBINDUNGEN AUS VERSCHIEDENEN CHEMISCHEN STOFFKLASSEN MITTELS DER ENZYME LACCASE UND MANGANPEROXIDASE**
BIOTRANSFORMATION OF BIOLOGICALLY ACTIVE COMPOUNDS MADE OF VARIOUS CLASSES OF CHEMICAL SUBSTANCES BY MEANS OF LACCASE AND MANGANESE PEROXIDASE ENZYMES
BIOTRANSFORMATION DE COMPOSES BIOLOGIQUEMENT ACTIFS PROVENANT DE CLASSES DE SUBSTANCES CHIMIQUES DIFFERENTES A L'AIDE DES ENZYMES LACCASES ET MANGANESE PEROXYDASES

(30) Priorität: 23.06.2000 DE 10029671
(43) Veröffentlichungstag der Anmeldung: 23.04.2003
(73) Patentinhaber: Dritte Patentportfolio Beteiligungsgesellschaft mbH & Co. KG, 12529 Schönefeld / Waltersdorf (DE)
(72) Erfinder: Jülich, Wolf Dieter, 17489 Greifswald (DE); Schauer, Frieder, 17493 Greifswald (DE); Lindequist, Ulrike, 17491 Greifswald (DE); Hammer, Elke, 17498 Wackerow (DE); Schäfer, Annett, 17498 Greifswald (DE); Jonas, Ulrike, 10405 Berlin (DE)
(74) Vertreter: Zech, Stefan Markus
(86) Internationale Anmeldenummer: PCT/EP2001/007152
(87) Internationale Veröffentlichungsnummer: WO 2001/098518

(56) Entgegenhaltungen:
- EP-A- 0 960 945
- BHALERAO UDAY T ET AL: "Laccase enzyme catalysed efficient synthesis of 3-substituted-1,2,4-triazolo(4,3-b)(4,1,2) -benzothiadiazine-8-ones." TETRAHEDRON, Bd. 50, Nr. 13, 1994, Seiten 4019-4024, XP002183145 ISSN: 0040-4020
- ANYANWUTAKU I ET AL: "Oxidative coupling of Mithramycin and Hydroquinone catalyzed by Copper Oxidases and Benzoquinone. Implications for the mechanism of action of aureolic acid antibiotics" BIOORGANIC & MEDICINAL CHEMISTRY, Bd. 2, Nr. 6, 1994, Seiten 543-551, XP001041413
- AGEMATU HITOSI ET AL: "Transformation of 7-(4-hydroxyphenylacetamido)cephalosporani c acid into a new cephalosporin antibiotic, 7-(1-oxaspiro(2.5)octa-6-oxo-4,7 -diene-2-carboxamido)cephalosporanic acid, by laccase." BIOSCIENCE BIOTECHNOLOGY AND BIOCHEMISTRY, Bd. 57, Nr. 8, 1993, Seiten 1387-1388, XP002183146 ISSN: 0916-8451
- OSIADACZ J ET AL: "On the use of Trametes versicolor laccase for the conversion of 4-methyl-3-hydroxyanthranilic acid to actinocin chromophore" JOURNAL OF BIOTECHNOLOGY, ELSEVIER SCIENCE PUBLISHERS, AMSTERDAM, NL, Bd. 72, Nr. 1-2, 11. Juni 1999 (1999-06-11), Seiten 141-149, XP004172894 ISSN: 0168-1656
- EGGERT CLAUDIA ET AL: "Laccase-mediated formation of the phenoxazinone derivative, cinnabarinic acid." FEBS LETTERS, Bd. 376, Nr. 3, 1995, Seiten 202-206, XP002183621 ISSN: 0014-5793
- BROWN R F; KINNICK M D; MORIN J M JR; VASILEFF R T; COUNTER F T ET AL.: "Synthesis and biological evaluation of a series of parenteral 3'-quaternary ammonium cephalosporins." JOURNAL OF MEDICINAL CHEMISTRY, Bd. 33, 1990, Seiten 2114-2121, XP001084083
- GIANFREDA L; XU F; BOLLAG J-M: "LACCASES: A USEFUL GROUP OF OXIDOREDUCTIVE ENZYMES" BIOREMEDIATION JOURNAL, Bd. 3, Nr. 1, 1999, Seiten 1-25, XP008017342
- AGEMATU, H. ET AL.: "Enzymatic Dimerization of Penicillin X" THE JOURNAL OF ANTIBIOTICS, Bd. 46, Nr. 1, Januar 1993 (1993-01), Seiten 141-148,
- D'ANNIBALE A; CELLETTI D; FELICI M; DI MATTIA E; GIOVANNOZZI-SERMANNI G: "Substrate specificity of laccase from Lentinus edodes" ACTA BIOTECHNOLOGICA, Bd. 16, Nr. 4, 1996, Seiten 257-270,
- EGGERT, C. ET AL.: "Laccase-mediated formation of the phenoxazinone derivative, cinnabarinic acid" FEBS LETTERS, Bd. 376, 1995, Seiten 202-206,
- AGEMATU ET AL.: "Transformation of 7-(4-hydroxyphenylacetamido)cephalosporani c acid into a new cephalosporin antibiotic, 7-(1-oxaspiro(2.5)octa-6-oxo-4,7 -diene-2-carboxamido)cephalosporanic acid, by laccase" BIOSCI. BIOTECH. BIOCHEM., Bd. 8, 1993, Seiten 1387-1388,

## Beschreibung

Offenbart werden neue biologisch aktive Verbindungen, Verfahren zu ihrer Herstellung und ihre Verwendung. Aus Antibiotika und Chemotherapeutika werden bei einer mit den Enzymen Laccase katalysierten Biotransformation Kopplungsprodukte erhältlich, die entweder eine verbesserte antimikrobielle Wirksamkeit oder verbesserte Anwendungseigenschaften aufweisen.

### Stand der Technik

Antibiotika bilden eine hinsichtlich ihrer Struktur und biologischen Wirkung heterologe Gruppe von Verbindungen, die zu den In der Human- und Veterinärmedizin sowie im Pflanzenschutz am meisten angewandten Therapeutika zählen. Zu den Antibiotika gehören von Mikroorganismen gebildete Stoffe sowie daraus gebildete Derivate, die bei intrasomatischer Anwendung Infektionskrankheiten bekämpfen können. Neben den antibakteriellen Antibiotika wurden Antibiotika mit antifungalen, antiviralen trypanoziden sowie mit kanzerostatischen Eigenschaften entdeckt. Durch die Anwendung von auf fermentativem Wege hergestellten Antibiotika In der Krebschemotherapie wurde die ursprüngliche Definition ebenso erweitert wie durch die Anwendung In der Tieraufzucht als Ergotropika und im Pflanzenschutz als Herbizide, Insektizide, Pestizide, Akarizide, Nematozide, Molluskizide (U.Gräfe: Biochemie der Antibiotika, Spektrum Akademischer Verlag, Heidelberg, Berlin, New York, 1992). Die Auffindung neuer zellulärer Wirkorte für Antibiotika und ähnliche bioaktive Wirkstoffe ist bis heute noch nicht abgeschlossen und führt zu neuen Anwendungsmöglichkeiten In der Human- und Veterinärmedizin sowie beispielsweise im Pflanzenschutz.

Die extensive partialsynthetische Abwandlung von natürlich vorkommenden Antibiotika und die chemische Totalsynthese haben schließlich zu Heilmitteln geführt, die das originäre Potential von Naturstoffen erheblich übertreffen. Schließlich wurden auch Totalsynthetika ohne Bezug auf mikrobielle Produkte aufgefunden, die hinsichtlich ihrer Wirkung auf Mikroorganismen und ihres Wirkungsmechanismus den klassischen Antibiotika gleichzusetzen sind (U.Gräfe: Biochemie der Antibiotika, Spektrum Akademischer Verlag, Heidelberg, Berlin, New York, 1992).

Im folgenden wird der Begriff Antibiotika in diesem umfassenden Sinne für biologisch aktive Wirkstoffe mit selektiven Wirkungen an unterschiedlichen Wirkorten von pathogenen Mikroorganismen (Bakterien, Pilze Viren) und phylogenetisch höherstehenden Organismen (z.B. Rickettsien, Trypanosomen, Tumorzellen oder Parasiten) gebraucht.

Antibiotika mit unterschiedlichen biologischen Aktivitäten für verschiedene Anwendungsgebiete werden ständig durch chemische Reaktionen modifiziert, um sie neuen Aufgabenstellungen anzupassen. Keine andere Gruppe von Antibiotika hat eine so umfangreiche halbsynthetische Modifikation erfahren wie die der β-Laktam-Antibiotika. Die Entdeckung der 6-Aminopenicillansäure als Grundkörper der Penicilline und der 7-Aminocephalosporansäure als Grundkörper der Chephalosporine leitete eine neue Etappe der Modifikation mit der Zielrichtung wirkungsoptimierter Derivate ein, die bis heute nicht abgeschlossen ist.

Die Vorteile der ursprünglich entdeckten Penicilline und Cephalosporine wie sehr geringe Toxizität und hohe bakterizide Wirkung, wurden durch ständige chemische Modifikation den steigenden Anforderungen angepasst. US-A-5,695,951 und US-A5,939,299 offenbaren Methoden, das Chephalosporin-Derivat Cephalexin mittels einer Haloperoxidase zu chlorieren. Eine Methode zur enzymatischen Einführung von Halogenen unter Nutzung von Haloperoxidasen wird In WO-A-00/15771 beschrieben.

Diese chemischen und biochemischen Modifikationen der β-Laktamantibiotika konnte jedoch mit der Resistenzentwicklung der pathogenen Mikroorganismen nicht Schritt halten, so dass heute zahlreiche Keime, Insbesondere mehrfachresiste Staphylokokken, nicht mehr mit den zur Zeit verfügbaren β-Laktamantibiotika therapiert werden können.

Laccasen (E.C. 1.10.3.2) sind Enzyme, die die Oxidation eines Substrates katalysieren. Sie werden aus Mikroben, Pflanzen und Tieren gewonnen. In der Natur spielen Laccasen und Peroxidasen z.B. beim biologischen Abbau von Lignin eine wichtige Rolle, deshalb können sie u.a. aus Weißfäulepilzen isoliert werden. Solche ligninolytischen Enzyme sind fähig, verschiedene Umweltschadstoffe oxidativ umzusetzen (Jonas, U, Hammer, E, Schauer F., Bollag, J.-M.: Biodegradation 1998; 8: 321-328; Bollag, J.-M., Shuttleworth, K.L., Anderson, D.H.: Appl Environ Microbiol 1988; 54: 3086-3091; Bumpus, J.A., Aust, S.D.: Bioessays 1986; 6: 166-170; Bumpus, J.A., Tien, M., Wright, D.A.: Science 1985; 228: 143-147).

DE-A-197 26 241 offenbart ein erweitertes Multikomponentensystem zur Abwasserbehandlung, das u.a. auch aus Weißfäulepilzen gewonnene Oxidoreductasen enthalten kann. Unter anderem kann auch Laccase In einer Konzentration von 0,001 bis 1 U/ml zur Abwasserbehandlung eingesetzt werden. Das für die Abwasserbehandlung entwickelte Multikomponentensystem kann auch für organische Synthesen z.B. zur Oxidation von ungesättigten Aliphaten oder zur Oxidation von Alkoholen zu Aldehyden eingesetzt werden. Als vorteilhaft hat sich nach DE-A-24 02 452 herausgestellt, durch Laccase vermittelte Oxidationen In einem zweiphasigen System aus Wasser und einem organischen Lösungsmittel vorzunehmen.

US-A-5,389,356 und US-A-5,468,628 beschreiben Methoden der selektiven Oxidation oder Reduktion, bei der eine Peroxidase als ein freie Radikale generierender Katalysator dient. Diese Methode ist z.B. geeignet, um Tetrachlorkohlenstoff abzubauen.

Es ist weiterhin bekannt, Phenole mittels Laccase oder Peroxidase zu oxidieren, wobei die Reaktionslösungen antimikrobiell wirksam sind und in industriellen Prozessen zur Keimbekämpfung eingesetzt werden können. Eine spezielle Anwendung der schonenden Oxidation mit Hilfe von Tyrosinasen und Laccasen wird in US-A-6,015,683 beschrieben. Dabei wird mit Hilfe der Laccase intermediär ein farbgebendes Reagenz hergestellt, das den spektrophotometrischen Nachweis von Acetaminophen In wässrigen Lösungen ermöglicht.

Auf der Oxidationskraft beruht auch die bekannte antimikrobielle Wirkung der Laccasen In Gegenwart von Sauerstoff. Ebenfalls auf einer Oxidation beruht die Bildung von antimikrobiell wirksamen Phenoxazinon-Derivaten unter dem Einfluß von Laccase. Laccase und Manganperoxidase werden bereits für die verschiedensten Zwecke wirtschaftlich genutzt. Sie bleichen ligninenthaltendes Material und werden deshalb in der Papierindustrie eingesetzt. Aus WO-A-95/01426 ist bekannt, dass organisch-chemische Verbindungen, die aus wenigstens zwei aromatischen Ringen bestehen, die Aktivität der Laccase verstärken können. Diese Verstärkung wurde ausgenutzt, um Farbstoffe in Lösungen oder Lignin und ligninenthaltende Materialien zu bleichen.

In US-A-4,478,683 wird beschrieben, das Wachstum von Mikroorganismen bei der industriellen Abwasseraufbereitung durch Zusatz von Laccasen und Peroxidasen zu verhindern.

Laccase und Manganperoxidase wurden in wenigen Fällen eingesetzt, um eine Kopplung verschiedener biologischer Verbindungen vorzunehmen. Da dabei vorzugsweise mit einem Angriff auf für die Wirksamkeit essentielle funktionelle Gruppen gerechnet werden muss, ist in der Regel eine Verschlechterung der Wirksamkeit zu erwarten. Wahrscheinlich aus diesem Grund beschränken sich Versuche zum Einsatz von Manganperoxidase und Laccase zur Derivatisierung von Antibiotika auf wenige Beispiele. Bei den bisher realisierten Beispielen wurde das angestrebte Ziel einer Verstärkung der antimikrobiellen Wirkung nicht erreicht. Zum Beispiel wurde Laccase benutzt, um Antibiotika der Aureolsäuregruppe mit Hydrochinonen zu verbinden (Anyanwutaku, I.O., Petrowski, R.J., Rosazza, I.P.: Bioorg. Med. Chem. 1994; 2: 543-551). Die biologische Aktivität der Ausgangsstoffe ging dabei verloren. Agematu et al. (Biosci. Biotechnol. Biochem. 57 (8), 1387-1388 (1993) beschreiben die Transformation von 7-(4-hydroxyphenylacetamido)-cephalosporinsäure durch eine Laccase katalysierte phenolische Oxidation. Auch hier war das Reaktionsprodukt weniger wirksam als das ursprüngliche Cephalosporin. Uemtsu et al. (Jpn. Kokai Tokkyo JP 05,163,281) setzen Oxidasen zur Oxidation der Cephalosporine ein.

Aus Penicillinmethylester werden unter dem Einfluss von Laccase unter bestimmten Bedingungen 3 verschiedene Typen von Dimeren erhalten, die alle biologisch Inaktiv sind (Agematu, H., Tsuchida, T., Kominato, K., Shibamoto, N., Yoshioka, T., Nishida, H., Okamoto, R., Shin, T., Murano, S.: J. Antibiot. Tokyo. 1993; 46: 141-148). Die enzymatische Synthese von Dimeren des Phenols und von Penicillin wird von Agemato (Baiosainsu to Indasutori 1996, 54, 715-718) zusammengefasst.

Bisher zur Modifikation von Antibiotika eingesetzte Enzyme wie Haloperoxidase besitzen ein eingeschränktes Substratspektrum. Die von Haloperoxidasen vermittelte Chlorierung ist nur in speziellen Fällen einsetzbar.

Wenn Mikroorganismen der Dauereinwirkung subletaler Antibiotika-Konzentration ausgesetzt sind, entwickeln sie als Teil ihres genetisch programmierten Anpassungsmechanismus an wechselnde Umweltbedingungen Antibiotika-resistenzen, d.h. sie werden durch die ursprüngliche minimale Hemmkonzentration des betreffenden Wirkstoffes nicht mehr inhibiert. Da die Einwirkung subletaler Dosen niemals vollständig zu vermeiden ist, entwickelt sich gegen jeden neuen antibiotischen Wirkstoff seitens pathogener Mikroorganismen eine Resistenz. Unter dem Einfluss von Peroxidasen oder Laccasen gebildete Wirkstoffe mit Wirksamkeit gegen resistent gewordene Keime wurden bisher nicht beschrieben.

Durch die zunehmende Reslstenzproblematik besteht nach wie vor ein großer Bedarf an Antibiotika, der durch die dem Stand der Technik entsprechenden Wirkstoffe nicht gedeckt wird.

Das gilt Insbesondere für den Einsatz von Antibiotika bei klinisch schwer beherrschbaren Infektionskrankheiten. Bei Infektionen mit grampositive Keimen sind z.Z. nur noch Glykopeptid-Antibiotika ausreichend wirksam. Grampositive Erreger wie Staphylokokken und Enterokokken entwickeln in zunehmendem Maße eine Mehrfachresistenz. Aber auch durch gramnegative Erreger verursachte Infektionskrankheiten werden in Zukunft klinisch nicht mehr sicher beherrscht werden können. Ebenso werden In der Veterinärmedizin Infektionskrankheiten von Nutztieren mit multiresistenten Staphylokokken immer bedrohlicher. Durch eine mögliche Übertragung resistent gewordener Keime auf den Menschen droht eine zusätzliche Gefahr.

Die der Erfindung zugrundeliegende Aufgabe bestand darin, biologisch aktive Wirkstoffe mittels Biotransformation weiterzuentwickeln und als Kopplungsprodukte mit anderen antimikrobiellen Wirkstoffen für eine arzneiliche Verwendung nutzbar zu machen. Der Erfindung liegt insbesondere die Aufgabe zugrunde, den durch die zunehmende Resistenzentwicklung von Bakterien gegenüber herkömmlichen Antibiotika bestehenden Handlungsbedarf, insbesondere bei der Therapie von Infektionskrankheiten, in der Human- und Veterinärmedizin, durch Entwicklung eines Biotransformationsverfahrens, mit dem bisher unbekannte Wirkstoffe aus bekannten antimikrobiellen Wirkstoffen gewonnen werden können, zu decken.

Diese Aufgabe wird erfindungsgemäß durch ein Verfahren gemäß den Ansprüchen gelöst.

Vorzugsweise sind die Substrate β-Lactam-Antibiotika.

Erfindungsgemäß können aus diesen antimikrobiellen Wirkstoffen durch Umsetzung nach einem Verfahren gemäß Anspruch 1 bis 5 Verbindungen erhalten werden, die dadurch gekennzeichnet sind, dass eine bei den Ausgangsstoffen nicht nachweisbare Wirksamkeit, vorzugsweise gegen multiresistente Keime erreicht wird. Die durch Umsetzung nach dem erfindungsgemäßen Verfahren erhältlichen Verbindungen können ferner dadurch gekennzeichnet sein; dass die Ausbildung von Resistenzen durch kovalente Verknüpfung von Molekülen mit unterschiedlichem Wirkungsmechanismus und unterschiedlichen molekularen Angriffsorten erschwert wird. Es ist aber auch erfindungsgemäß, durch Einführung zusätzlicher hydrophiler und/oder hydrophober Komponenten eine Verbesserung der Anwendungseigenschaften zu erreichen, ohne das Wirkungsspektrum zu verändern.

Erfindungsgemäß eingesetzt werden vorzugsweise Enzyme, die der Klassifikation gemäß Internationaler Enzym-Nomenklatur (Enzym Nomenclature, Academic Press, Inc, 1992, S. 24-154) E C 1.10.3.2. (Laccase).

Wird das erfindungsgemäße Verfahren in einer wässrigen Lösung durchgeführt, weist diese vorzugsweise einen pH Wert von pH 2-8, vorzugsweise bei pH 3-5, sowie Temperaturen zwischen 5°C und 60°C auf.

Als polyfunktionelle Synthone kommen in Frage: C1-C18 -Alkyl-; C1-C18 -Alkenyl-; C1-C18 -Alkynyl-; C1-C18 -Alkoxy-; C1-C18 -Oxycarbonyl-; C1-C18 -Oxoalkyl-; C1-C18 -Alkylsulfanyl-; C1-C18 - Alkylsulfonyl-; C1-C18 -Alkylimino- oder Alkylamino-Synthone. Bevorzugt sind erfindungsgemäß die aromatischen Moleküle im folgenden: mono-, di- oder polycyclische aromatische Synthone, jeweils optional mit einer oder mehreren funktionellen Gruppen oder Substituenten aus der Gruppe der Halogene; Sulfo-; Sulfon-; Sulfamino; Sulfanyl-; Amino-; Amido-; Nitro-; Azo-; Imino-; Carboxy-; Cyano-; Formyl-; Hydroxy-; Halocarbonyl-; Carbamyl-; Carbamidoyl-; Phosphon-; Phosphonyl-; C1-18 -Alkyl-; C1-18 -Alkenyl- ; C1-18 -Alkinyl-; C1-18 -Alkoxy-; C1-18 -Oxycarbonyl-; C1-18 -Oxoalkyl-; C1-18 -Alkylsulfanyl-; C1-18 -Alkylsulfonyl-; C1-18 -Alkylimino- oder Alkylamino-Substituenten ausgestattet. Beispiele für solche Verbindungen sind ohne andere auszuschließen , Aminophenole, Phenole, sowie 2,5-dihydroxylierte Benzoesäurederivate. Weiterhin kommen ohne andere auszuschließen folgende Verbindungen in Betracht: 3,4-Diethoxyanilin, 2-Methoxy-p-phenylendiamin, 1-Amino-4-betamethoxyethyl-aminobenzen, (N-.beta.-Methoxyethyl-p-phenylendiamin), 1-Amino-4-bis-(.beta.-hydroxy-ethyl)-aminobenzen, 2-Methyl-1,3-dlaminobenzen (2,6-Diaminotoluen), 2,4-Diaminotoluen, 1-Amino-4-sulfonsäurebenzen, 1-N-Methylsulfonsäure-4-aminobenzen, 1-Methyl-2-hydroxy-4-amino-benzen (3-Amino-o-cresol), 1-Methoxy-2,4-diaminobenzene (2,4-Diaminoanisole), 1-Ethoxy-2,3-diamino-benzene (2,4-Diaminophenetole), 1-beta-Hydroxyethyloxy-2,4-diamino-benzen (2,4-Diaminophenoxyethanol), 1,3-Dihydroxy-2-methylbenzene (2-Methylresorcinol), 1,2,4-Trihydroxybenzen, 1,2,4-Trihydroxy-5-methylbenzen (2,4,5-Trihydroxytoluen), 2,3,5-Trihydroxytoluen, , 1,4-Phenylenediamin, 2,5-Diaminotoluen, 2-Chloro-1,4-phenylenediamin, 2-Aminophenol, 3-Aminophenol, 4-Aminophenol, 1,3-Phenylenediamin, , 1,2,3-Benzentriol (Pyrogallol), 1,3-Benzendiol (Resorcinol), 1,2-Benzendiol (Pyrocatechol), 2-Hydroxyzimtsäure, 3-Hydroxyzimtsäure, 4-Hydroxyzimtsäure, 2,3-Diaminobenzoesäure, 2,4-Diaminobenzoesäure, 3,4-Diaminobenzoesäure, 3,5-Diaminobenzoesäure, Methyl-2,3-diaminobenzoat, Ethyl-2,3-diaminobenzoat, Isopropyl-2,3-diaminobenzoat, Methyl-2,4-diaminobenzoat, Ethyl-2,4-diaminobenzoat, Isopropyl-2,4-diaminobenzoat, Methyl-3,4-diaminobenzoat, Ethyl-3,4-diaminobenzoat, Isopropyl-3,4-diaminobenzoat, Methyl-3,5-diaminobenzoat, Ethyl-3,5-diaminobenzoat, Isopropyl-3,5-diaminobenzoat, N,N-Dlethyl-3,4-diaminobenzoesäureamid, N,N-Dipropyl-3,4-diaminobenzoesäureamid, 3,4-Dihydroxybenzaldehyd, Anthranilsäure, 4-Aminobenzoesäure, Ethylhydrochinon, Mandelsäure, o-Nitrobenzaldehyd, Benzylimidazol, Salicylsäure, 3-Aminosalicylsäure, 4-Aminosalicylsäure, 5-Aminosalicylsäure, Methyl-3-aminosalicylat, Methyl-4-aminosalicylat, Methyl-5-amlnosalicylat, Ethyl-3-aminosalicylat, Ethyl-4-aminosalicylat, Ethyl-5-aminosalicylat, Propyl-3-aminosalicylat, Propyl-4-aminosalicylat, Propyl-5-aminosalicylat, Salicylsäureamid, 4-Aminothiophenol, 4-Hydroxythiophenol, N,N-Dimethyl-1,4-phenylendiamin, N,N-Diethyl-1,4-phenylendiamin, N-Phenyl-1,2-phenylendiamin, 1,2-Phenylendiamin, 2-Nitroanilin, 3-Nitroanilin, 2-Chloranilin, 3-Chloranilin, 4-Chloranilin, 4-Aminoacetanilid.

Im erfindungsgemäßen Verfahren kann durch Einsatz rekombinanter Laccasen eine Veränderung des pH-Optimums erreicht wird.

Die Kopplungsreaktion kann erfindungsgemäß ggf. in Gegenwart mindestens eines Mediators aus der Gruppe der Hydroxylamine und/oder Hydroxamsäuren und ggf. eines Mediators aus der Gruppe der Amide stattfinden.

Gegebenenfalls können die Reaktionsprodukte nach Beendigung der Reaktion stabilisiert werden.

Offenbart werden auch Verbindungen, die aus Wirkstoffen verschiedener Stoffklassen durch eine Biotransformation gemäß der Erfindung erhältlich sind.

Die Offenbarten biologisch aktiven Verbindungen können in der Human- und Veterinärmedizin sowie im Pflanzenschutz als alleiniger Wirkstoff oder in Form von Kombinationspräparaten Verwendung finden.

Die Verwendung der Offenbarten biologisch aktiven Verbindungen können insbesondere bei der Bekämpfung von Infektionskrankheiten, vorzugsweise als Mittel mit antimikrobieller und antiviraler Wirkung zur lokalen und/oder systemischen Anwendung, bei Infektionskrankheiten mit multiresistenten grampositiven oder gramnegativen Keimen, als Antimykotikum zur lokalen und/oder systemischen Anwendung sowie als Zytostatikum in der Human- und Veterinärmedizin eingesetzt werden.

Offenbarten sind sind auch Arzneimittel enthaltend eine oder mehrere der Offenbarten Verbindungen.

Die nach der Blotransformation erhaltenen Verbindungen können allein sowie in Kombination untereinander eingesetzt werden.

Durch Einführung zusätzlicher hydrophiler und/oder hydrophober Komponenten kann erfindungsgemäß eine Verbesserung der Anwendungseigenschaften der Ausgangsverbindungen erreicht werden. Die Offenbarten Verbindungen können insbesondere als grenzflächenaktive Stoffe eingesetzt werden.

Die Offenbarten Verbindungen, die aus antimikrobiellen Wirkstoffen durch Umsetzung nach dem erfindungsgemäßen Verfahren erhältlich sind, zeigen vorteilhafterweise eine bei den Ausgangsstoffen nicht nachweisbare Wirksamkeit, vorzugsweise gegen multiresistente Keime.

Die Offenbarten Verbindungen, die aus antimikrobiellen Wirkstoffen durch Umsetzung nach dem erfindungsgemäßen Verfahren erhältlich sind, erschweren die Ausbildung von Resistenzen durch die durch Einwirkung von radikalbildenden Enzymen erreichte kovalente Verknüpfung von Molekülen mit unterschiedlichem Wirkungsmechanismus und unterschiedlichen molekularen Angriffsorten.

Aufbauend auf einer durch Laccasen sowie durch Laccase ähnliche radikalbildende Enzyme katalysierten Kopplungsreaktion wird erfindungsgemäß ein allgemein anwendbares Verfahren der Biotransformation zugänglich, mit dem verschiedene biologisch aktive Wirkstoffe, die als funktionelle Gruppe Amino- oder Hydroxylgruppen enthalten, so miteinander verbunden werden können, dass eine Verbesserung der biologischen Aktivität und/oder eine Verbesserung der Anwendungseigenschaften erreicht wird. Wesentliche kennzeichnende Schritte zur Herstellung der neuen Verbindungen sind enzymatisch katalysierte Ein-Elektronen-Oxidationen, eine enzymatisch katalysierte Bildung des Superoxidradikals oder eine enzymatisch katalysierte Bildung des Arylradikals.

Überraschend wurde gefunden, dass durch diese enzymatisch katalysierten Umsetzungen Wirkstoffe mit einer biologischen Aktivität erhältlich werden, die den Ausgangsstoffen deutlich überlegen ist. Für diese enzymatisch katalysierten Reaktionsschritte Ist erfindungsgemäß die Anwesenheit von insbesondere Laccase erforderlich. In diesem Zusammenhang werden unter Laccasen insbesondere die unter der Klassifikation EC 1. 10.3.2. zusammengefassten Enzyme verstanden. Die Laccasen werden vorzugsweise aus Pilzen der Gattungen Pycnoporus, Trametes, Coriolus, Collybia, Fomes, Lentinus, Pleurotus, Rhizoctonia, Aspergillus, Neurospora, Podospora, Phlebia oder Myceliophthora oder auf biotechnologischem Weg gewonnen.

Mit dem erfindungsgemäßen Verfahren können Wirkstoffe aus verschiedenen chemischen Stoffklassen miteinander verbunden werden. Vorteilhafterweise weisen die Wirkstoffe, die miteinander verbunden werden sollen, biologische Wirkungen mit verschiedenen Angriffspunkten auf. Es ist jedoch auch möglich, mit dem erfindungsgemäßen Verfahren einen biologisch aktiven Wirkstoff mit einer Substanz ohne antimikrobielle Eigenwirkung zu verbinden, um die Anwendungseigenschaften, z.B. das Verteilungsverhalten, zu verbessern. Durch die erfindungsgemäße Einwirkung von Laccase auf antimikrobiell wirksame Ausgangsstoffe werden neue biologisch aktive Verbindungen erhalten, die verbesserte Anwendungseigenschaften aufweisen. Es war völlig überraschend, dass sich Insbesondere β-Laktamantibiotika wie Penicilline, Cephalosporine und Carbapeneme mit dem erfindungsgemäßen Verfahren unter Erhalt der antimikrobiellen Wirksamkeit umsetzen lassen. Besonders vorteilhaft ist es, β-Laktam-durch eine laccasekatalysierte Reaktion mit chinoiden und hydrochinoiden Wirkstoffen zu verbinden. Die durch die erfindungsgemäße Biotransformation erhältlichen neuen Wirkstoffe zeigen eine antimikrobielle Wirksamkeit selbst gegen multiresistente Keime. Damit werden die Voraussetzungen für eine Anwendung der neuen Wirkstoffe In der Human- und Veterinärmedizin zur Bekämpfung von Infektionskrankheiten geschaffen. Die mit dem erfindungsgemäßen Verfahren erhältlichen neuen Wirkstoffe können sowohl lokal als auch systemisch, allein sowie in Form von Kombinationspräparaten eingesetzt werden.

### Beispiel 1

Herstellung neuer antimikrobieller Wirkstoffe durch Kopplung von 2,5-Dihydroxy-N-(2-hydroxyethyl)benzamid mit 6-Aminopenicillansäure mittels

### Biotransformation

### Methodik:

### 1.1. Herstellung der Enzymlösung

### Kultivierung der filamentösen Pilze

Die Kultivierung der filamentösen Pilze erfolgte zuerst auf Schrägagar (Malzagar) bei 30°C 7 d und dann 7 d auf Malzagarplatten bei 30°C. Drei ca. 1 cm² große, gut bewachsene Malzagarstücke wurden anschließend aus einer Agarplattenkultur mit einem sterilen Spatel ausgeschnitten und in 300-ml-Erlenmeyer-Kolben mit 60 ml BSM übertragen. Diese Vorkultur wurde bei 30°C über 7 Tage als Standkultur inkubiert.

Das bei den Glucosevorkulturen der filamentösen Pilze gebildete Myzel wurde mit Hilfe eines Ultraturraxgerätes bei 17000 U/min dreimal 10 s homogenisiert. 2,5 ml des Homogenisats wurden für die Experimente In 100-ml-ErlenmeyerKolben mit 25 ml BSM überimpft. Kolben und Medium wurden getrennt voneinander sterilisiert, um Medienverluste bei der Hitzesterilisation im Autoklaven zu vermeiden. Vor der Beimpfung wurde das Medium in die Kolben gefüllt. Diese Ansätze wurden mit einer 0,05 %igen sterilfiltrierten Tween 80-Lösung supplementiert. Die Kolben wurden In einem Schüttelinkubator bei 158 U/min und 30°C kultiviert.

### Anionenaustauscherchromatographie

Zur verstärkten Ausscheidung der ligninolytischen Enzyme In Kulturen von T. *versicolor* und *Pycnoporus cinnabarinus* wurde Veratrylalkohol eingesetzt. Da diese Verbindung und ihre Metaboliten bei späteren Inkubationsexperimenten mit Kulturfiltrat störend wirken, erfolgte ihre Abtrennung mit Q-Sepharose. Dazu wurde 1 ml der Anionenaustauschermatrix mit 100 ml Histidinpuffer dreimal equilibriert. Anschließend wurde die equilibrierte Q-Sepharose mit 80 ml Kulturfiltrat 1 h langsam auf einem Magnetrührer bei Raumtemperatur gerührt. Danach wurde der wässrige Überstand von der Q-Sepharose durch GF-6 Glasphaserfilter abfiltriert, die Matrix mit je 10 ml Histidinpuffer 20 mal gewaschen und das Protein mit 15 ml Hochsalz-Histidinpuffer eluiert.

### Ultrafiltration

Zur Vergleichbarkeit der Experimente mit Kulturfiltrat sollte In den Inkubationsansätzen mit gleicher Enzymaktivität gearbeitet werden. Dazu war es notwendig, die gereinigten Kulturüberstände einzuengen und für die Umsätze entsprechend zu verdünnen. Die Konzentration erfolgte durch Ultrafiltration mit Centricon-30 und Centriprep-30 Mikrokonzentratoren in einer Kühlzentrifuge mit 3000 U/min bei 4°C für 60 min.

### Entsalzung

Für die Umsätze mit Kulturfiltrat war es erforderlich, die gereinigte und eingeengte Proteinprobe zu entsalzen. Auf eine gepackte Sephadex G-25 Superfine Säule wurden 2,5 ml Probe aufgetragen und mit 3,7 ml Histidinpuffer eluiert.

Das erhaltene Proteineluat wurde bei -20°C gelagert.

### 1.2. Kopplungsreaktion

Das 2,5-Dihydroxy-N-(2-hydroxyethyl)benzamid wird im äquimolaren Verhältnis mit 6-Aminopenicillansäure (1 mM) In Natriumacetatpuffer (pH 5; 0,02 M) unter dem Einfluss einer Laccase (975 nmol/2 mi/min; gewonnen aus *Trametes versicolor*) 35 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochletstungsflüssigchromatographie gereinigt.

Ergebnis: Eine Kopplung des Antibiotikagrundkörpers 6-Aminopenicillansäure mit dem 2,5-Dihydroxy-N-(2-hydroxyethyl)benzamid führt zu einer antimikrobiell wirksamen Verbindung. Die Ausbeute an Kopplungsprodukt beträgt 66%.

Die Analyse der erhaltenen Verbindung mittels massenspektrometrischer Methoden ergab eine Masse von 411.

In der Summe der Ergebnisse zur Strukturanalyse ist für das erhaltene Kopplungsprodukt die folgende Struktur anzunehmen:

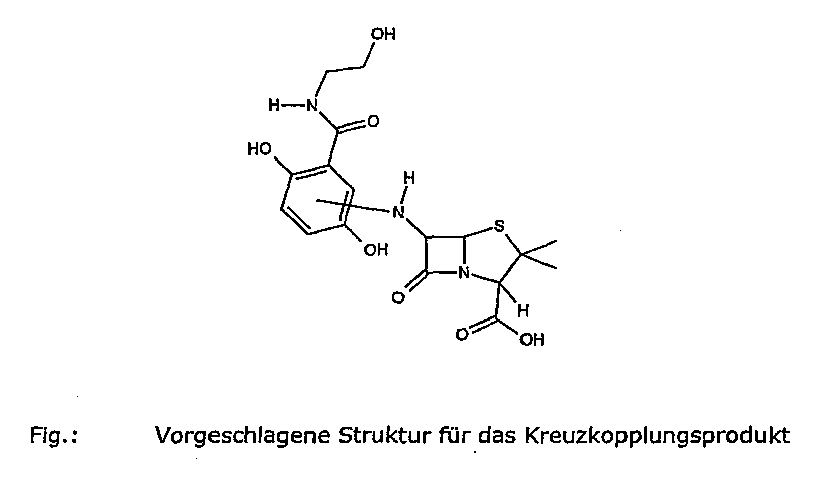

### Beispiel 2

Herstellung neuer antimikrobieller Wirkstoffe durch Kopplung von 2,5-Dihydroxy-N-(2-hydroxyethyl)benzamid mit 7-Aminocephalosporinsäure mittels Biotransformation

Methodik: Die Herstellung der Enzymlösung erfolgte nach Beispiel 1.

Das 2,5-Dihydroxy-N-(2-hydroxyethyl)benzamld wird im äquimolaren Verhältnis mit 7-Aminocephalosporinsäure (1 mM) In Natriumacetatpuffer (pH 5; 0,02 M) unter dem Einfluss einer Laccase (975 nmol/2 ml/min; gewonnen aus *Pycnoporus cinnabarinus)* 35 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographie gereinigt.

Ergebnis: Eine Kopplung des Antibiotikagrundkörpers 7-Aminocephalosporinsäure mit dem 2,5-Dihydroxy-N-(2-hydroxyethyl)benzamids führt zu einer bisher unbekannten antimikrobiell wirksamen Verbindung. Die Ausbeute an Kopplungsprodukt beträgt 68%.

### Beispiel 3

Herstellung neuer antimikrobieller Wirkstoffe durch Kopplung von 2,5-Dihydroxy-N-(2-hydroxyethyl)benzamid mit 7-Aminodesacetoxycephalosporinsäure mittels Biotransformation

Methodik: Die Herstellung der Enzymlösung erfolgte nach Beispiel 1.

Das 2,5-Dihydroxy-N-(2-hydroxyethyl)benzamid wird im äquimolaren Verhältnis mit 7-Aminodesacetoxycephalosporinsäure (1 mM) In Natriumacetatpuffer (pH 5; 0,02 M) unter dem Einfluss einer Laccase (975 nmol/2 ml/min; gewonnen aus *Trametes versicolor*) 40 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat. Der nach der Entfernung des Lösungsmittels verbleibende Rückstand wird mit Hochleistungsflüssigchromatographie gereinigt.

Ergebnis: Eine Kopplung des Antibiotikagrundkörpers 7-Aminodesacetoxycephalosporinsäure mit dem 2,5-Dihydroxy-N-(2-hydroxyethyl)benzamids führt zu einer bisher unbekannten antimikrobiell wirksamen Verbindung. Die Ausbeute an Kopplungsprodukt beträgt 73%.

Die Analyse der erhaltenen Verbindung mittels massenspektrometrischer Methoden ergab eine Masse von 409. Die Ergebnisse der kernresonanzspektroskopischen Analysen werden In der folgenden Tabelle zusammengefasst. Zum Vergleich der ¹H-NMR-Signale wurde auch das 2,5-Dihydroxy-N-(2-hydroxyethyl)benzamid untersucht. Die Messungen erfolgten im Lösungsmittel DMSO-d₆ mit dem internen Standard TMS mit einem ARX 300 (Bruker, Karlsruhe).

**Tabelle: ¹H-NMR-Spektrenvergleich des Kreuzkopplungsproduktes 3 mit der Ausgangsverbindung 2,5-Dihydroxy-N-(2-hydroxyethyl)benzamid**

| Resonanzen [ppm] Kreuzkopplu ngs-produkt 3 | Multiplizität, Kopplungskonstante | Resonanzen [ppm] 2,5-Dihydroxy-N-(2-hydroxyethyl)-benzamid | Multiplizität, Kopplungskonstante | Atomanzahl und -art, Zuordnung |
|---|---|---|---|---|
| 12,79 | s | - | - | 1H, COOH |
| 9,98 | s | 11,60 | s, | 1H, OH am C2, C₆H₂₍₃₎ |
| 9,66 | s | 8,98 | s | 1H, OH am C5, C₆H₂₍₃₎ |
| 8,50 | t, ³J = 5,3 | 8,69 | t, ³J = 5,3 | 1H, NH, CH₂-NH |
| 6,73 | s | 7,27 | d(s), ⁴J = 2,8 | 1H, H am C6, C₆H₂₍₃₎ |
| 6,65 | s | 6,85 | dd, ³J = 8,7, ⁴J = 2,8 | 1H, H am C4, C₆H₂₍₃₎ |
| - | | 6,73 | d, ³J = 8,7 | 1H, H am C3, C₆H₂₍₃₎ |
| 6,42 | d, ³J = 9,3 | - | | 1H, NH am β-Lactamring |
| 5,85 | t, ³J = 5,4 | 4,78 | t, ³J = 5,5 | 1H, OH, CH₂-OH |
| 5,48 | m, ³J = 9,3, ³J = -4,4 | | | 1H, H7 β-Lactamring |
| 5,02 | m, ³J = 4,4 | - | | 1H, H6 β-Lactamring |
| 3,63 | m | 3,54 | m | 2H, CH₂, CH₂-OH |
| 3,49 | m | 3,36 | m | 2H, CH₂, CH₂-NH |
| 3,29/3,50 | ABq, ²J = 17,2 | - | | 2H, CH₂, heterocyclischer Ring |
| 1,91 | s | - | | 3H, CH₃ |

In der Summe der Ergebnisse zur Strukturanalyse Ist für das erhaltene Kopplungsprodukt die folgende Struktur anzunehmen:

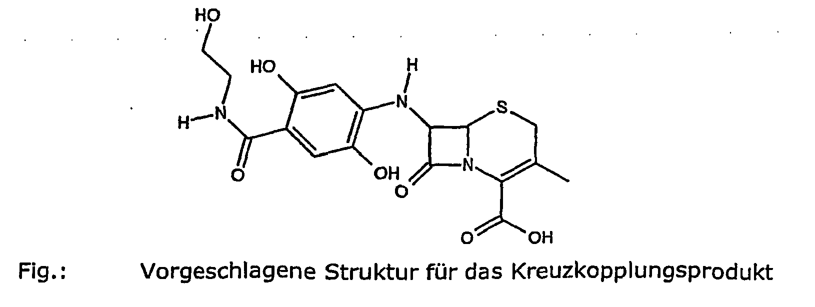

### Beispiel 4

### Herstellung neuer antimikrobieller Wirkstoffe durch Kopplung von 2,5-Dihydroxy-N-(2-hydroxyethyl)benzamid mit Amoxicillin mittels Biotransformation

Methodik: Die Herstellung der Enzymlösung erfolgte nach Beispiel 1.

Das 2,5-Dihydroxy-N-(2-hydroxyethyl)benzamid wird im äquimolaren Verhältnis mit Amoxicillin (1 mM) in Natriumacetatpuffer (pH 5; 0,02 M) unter dem Einfluss einer Laccase (975 nmol/2 ml/min; gewonnen aus *Trametes versicolor)* 50 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographle gereinigt.

Ergebnis: Eine Kopplung des Antibiotikums Amoxicillin mit dem 2,5-Dihydroxy-N-(2-hydroxyethyl)benzamids führt zu einer bisher unbekannten antimikrobiell wirksamen Verbindung. Die Ausbeute an Kopplungsprodukt beträgt 55%.

Die Analyse der erhaltenen Verbindung mittels massenspektrometrischer Methoden ergab eine Masse von 560. Die Ergebnisse der kernresonanzspektroskopischen Analysen werden in der folgenden Tabelle zusammengefaßt. Zum Vergleich der ¹H-NMR-Signale wurden auch das 2,5-Dihydroxy-N-(2-hydroxyethyl)benzamid (siehe Tabelle zu Beispiel 3) und Amoxicillin untersucht. Die Messungen erfolgten im Lösungsmittel CD₃OD mit dem Internen Standard TMS mit einem ARX 300 (Bruker, Karlsruhe).

**Tabelle: ¹H-NMR-Spektrenvergleich des Kreuzkopplungsproduktes 4 mit der Ausgangsverbindung Amoxicillin**

| Resonanzen [ppm] Kreuzkopplu ngs-produkt 4 | Multiplizität, | Resonanzen [ppm] Amoxicillin | Multiplizität, | Atomanzahl und -art, Zuordnung |
|---|---|---|---|---|
| 7,48 | d, ³J = 7,2 | 7,49 | d, ³J = 7,2 | 2H, H am C3/C5, C₆H₄ |
| 6,85 | d, ³J = 7,2 | 6,83 | d, ³J = 7,2 | 2H, H am C2/C6, C₆H₄ |
| 6,74 | d, ³J = 8,6 | - | - | 1H, H am C3, C₆H₂ |
| 6,63 | d, ³J = 8,6 | - | - | 1H, H am C4, C₆H₂ |
| 5,99 | m, ³J = 4,2 | 5,99 | m, ³J = 4,2 | 1H, H7 β-Lactamring |
| 5,92 | m, ³J = 4,2 | 5,92 | m, ³J = 4,2 | 1H, H6 β-Lactamring |
| 4,31 | s | 4,31 | s | 1H, CH, heterocyclischer Ring |
| 3,70 | m | - | - | 2H, CH₂, CH₂-OH |
| 3,49 | m | - | - | 2H, CH₂, CH₂-NH |
| 1,57 | s | 1,57 | s | 3H, CH₃, heterocyclischer Ring |
| 1,50 | s | 1,50 | s | 3H, CH₃, heterocyclischer Ring |

In der Summe der Ergebnisse zur Strukturanalyse ist für das erhaltene Kopplungsprodukt die folgende Struktur anzunehmen:

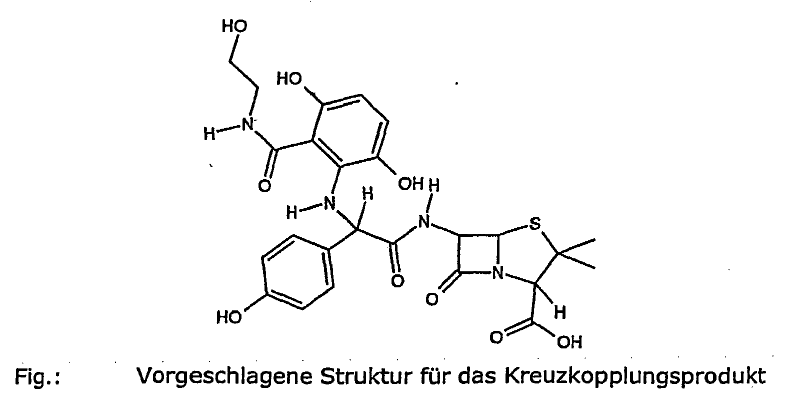

### Beispiel 5

Herstellung neuer antimikrobieller Wirkstoffe durch Kopplung von 2,5-Dihydroxy-N-(2-hydroxyethyl)benzamid mit Ampicillin mittels Biotransformation

### Methodik: Die Herstellung der Enzymlösung erfolgte nach Beispiel 1.

Das 2,5-Dihydroxy-N-(2-hydroxyethyl)benzamid wird im äquimolaren Verhältnis mit Ampicillin (1 mM) in Natriumacetatpuffer (pH 5; 0,02 M) unter dem Einfluss einer Laccase (975 nmol/2 ml/min; gewonnen aus *Trametes versicolor*) 50 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hoch-Ieistungsfiüssigchromatographie gereinigt.

Ergebnis: Eine Kopplung des Antibiotikums Ampicillin mit dem 2,5-Dihydroxy-N-(2-hydroxyethyl)benzamids führt zu einer bisher unbekannten antimikrobiell wirksamen Verbindung. Die Ausbeute an Kopplungsprodukt beträgt 75%.

Die Analyse der erhaltenen Verbindung mittels massenspektrometrischer Methoden ergab eine Masse von 544.

In der Summe der Ergebnisse zur Strukturanalyse ist für das erhaltene Kopplungsprodukt die folgende Struktur anzunehmen: 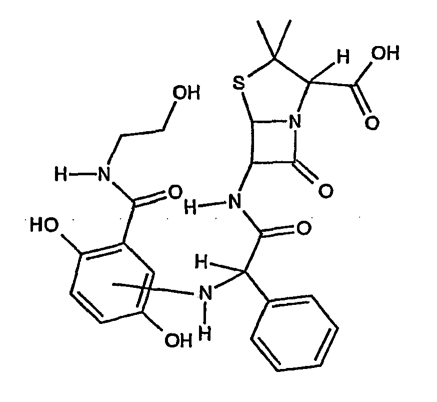 Fig.: Vorgeschlagene Struktur für das Kreuzkopplungsprodukt

### Beispiel 6

### Herstellung neuer antimikrobieller Wirkstoffe durch Kopplung von 2,5-Dihydroxy-N-(2-hydroxyethyl)benzamid mit Sulfonamiden mittels Biotransformation

Das 2,5-Dihydroxy-N-(2-hydroxyethyl)benzamid wird im äquimolaren Verhältnis mit p-Aminobenzensulfonamid (1 mM) In Natriumacetatpuffer (pH 5; 0,02 M) unter dem Einfluß einer Laccase (975 nmol/2 ml/min; gewonnen aus *Pycnoporus cinnabarinus*) 30 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographie gereinigt.

Ergebnis: Eine Kopplung des biologisch aktiven Wirkstoffes p-Aminobenzensulfonamid mit dem 2,5-Dihydroxy-N-(2-hydroxyethyl)benzamids führt zu einer bisher unbekannten antimikrobiell wirksamen Verbindung. Die Ausbeute an Kopplungsprodukt beträgt 74%.

### Beispiel 7

### Herstellung neuer antimikrobieller Wirkstoffe durch Kopplung von 2,5-Dihydroxy-N-(2-hydroxyethyl)benzamid mit substituierten Salicylsäuren mittels Biotransformation

Das 2,5-Dihydroxy-N-(2-hydroxyethyl)benzamid wird im äquimolaren Verhältnis mit 4-Amino-2-hydroxybenzoesäure (1 mM) In Natriumacetatpuffer (pH 5; 0,02 M) unter dem Einfluß einer Laccase (975 nmol/2 ml/min; gewonnen aus z.B. *Trametes versicolor* oder *Pycnoporus cinnabarinus*) 50 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographie gereinigt.

Ergebnis: Eine Kopplung der 4-Amino-2-hydroxybenzoesäure mit dem 2,5-Dihydroxy-N-(2-hydroxyethyl)benzamlds führt zu einer bisher unbekannten antimikrobiell wirksamen Verbindung. Die Ausbeute an Kopplungsprodukt beträgt 73%.

### Beispiel 8

### Herstellung neuer antimikrobieller Wirkstoffe durch Kopplung von 2,5-Dihydroxybenzoesäuren und -estern mit 6-Aminopenicillansäure mittels Biotransformation

Methodik: Die Herstellung der Enzymlösung erfolgte nach Beispiel 1.

Der 2,5-Dihydroxybenzoesäuremethylester wird im äquimolaren Verhältnis mit 6-Aminopenicillansäure (1 mM) in Natriumacetatpuffer (pH 5; 0,02 M) unter dem Einfluss einer Laccase (975 nmol/2 ml/min; gewonnen aus *Pycnoporus cinnabarinus*) 1 h bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographie gereinigt.

Ergebnis: Eine Kopplung des Antibiotikagrundkörpers 6-Aminopenicillansäure mit dem 2,5-Dihydroxybenzoesäuremethylester führt zu einer bisher unbekannten antimikrobiell wirksamen Verbindung mit hoher Aktivität. Die Ausbeute an Kopplungsprodukt beträgt 66%.

### Beispiel 9

### Herstellung neuer antimlkrobieller Wirkstoffe durch Kopplung von 2,5-Dihydroxybenzoesäuren und -estern mit 7-Aminocephalosporinsäure mittels Biotransformation

Methodik: Die Herstellung der Enzymlösung erfolgte nach Beispiel 1.

Der 2,5-Dihydroxybenzoesäuremethylester wird im äquimolaren Verhältnis mit 7-Aminocephalosporinsäure (1 mM) In Natriumacetatpuffer (pH 5; 0,02 M) unter dem Einfluss einer Laccase (975 nmol/2 ml/min; gewonnen aus z.B. *Trametes versicolor)* 1 h bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographie gereinigt.

Ergebnis: Eine Kopplung des Antibiotikagrundkörpers 7-Aminocephalosporinsäure mit dem 2,5-Dihydroxybenzoesäuremethylester führt zu einer bisher unbekannten antimikrobiell wirksamen Verbindung. Die Ausbeute an Kopplungsprodukt beträgt 63%.

### Beispiel 10

### Herstellung neuer antimikrobieller Wirkstoffe durch Kopplung von 2,5-Dihydroxybenzoesäuren und -estern mit 7-Aminodesacetoxycephalosporinsäure mittels Biotransformation

Methodik: Die Herstellung der Enzymlösung erfolgte nach Beispiel 1.

Der 2,5-Dihydroxybenzoesäuremethylester wird im äquimolaren Verhältnis mit 7-Aminodesacetoxycephalosporinsäure (1 mM) In Natriumacetatpuffer (pH 5; 0,02 M) unter dem Einfluss einer Laccase (975 nmol/2 ml/min; gewonnen aus *Pycnoporus cinnabarinus*) 1 h bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographie gereinigt.

Ergebnis: Eine Kopplung von 2,5-Dihydroxybenzoesäuren und -estern mit 7-Aminodesacetoxycephalosporinsäure führt zu einer bisher unbekannten antimikrobiell wirksamen Verbindung. Die Ausbeute an Kopplungsprodukt beträgt 71%.

### Beispiel 11

### Herstellung neuer antimikrobieller Wirkstoffe durch Kopplung von 2,5-Dihydroxybenzoesäuren und -estern mit Amoxicillin mittels Biotransformation

Methodik: Die Herstellung der Enzymlösung erfolgte nach Beispiel 1.

Der 2,5-Dihydroxybenzoesäuremethylester wird im äquimolaren Verhältnis mit Amoxicillin (1 mM) in Natriumacetatpuffer (pH 5; 0,02 M) unter dem Einfluss einer Laccase (975 nmol/2 mi/min; gewonnen aus *Trametes versicolor*) 45 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographie gereinigt.

Ergebnis: Eine Kopplung des Antibiotikums Amoxicillin mit dem 2,5-Dihydroxybenzoesäuremethylester führt zu einer bisher unbekannten antimikrobiell wirksamen Verbindung. Die Ausbeute an Kopplungsprodukt beträgt 66%.

In der Summe der Ergebnisse zur Strukturanalyse ist für das erhaltene Kopplungsprodukt die folgende Struktur anzunehmen:

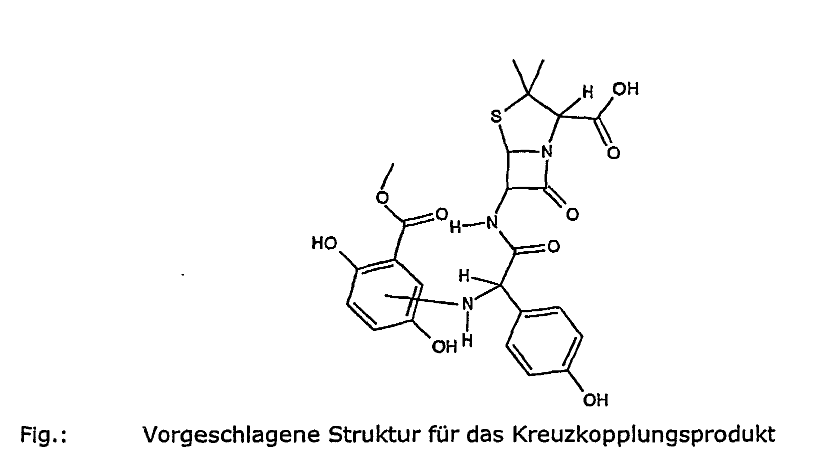

### Beispiel 12

### Herstellung neuer antimikrobieller Wirkstoffe durch Kopplung von 2,5-Dihydroxybenzoesäuren und -estern mit Ampicillin mittels Biotransformation

Methodik: Die Herstellung der Enzymlösung erfolgte nach Beispiel 1.

Der 2,5-Dihydroxybenzoesäuremethylester wird im äquimolaren Verhältnis mit Ampicillin (1 mM) In Natriumacetatpuffer (pH 5; 0,02 M) unter dem Einfluss einer Laccase (975 nmol/2 ml/min; gewonnen aus *Trametes versicolor*) 45 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographie gereinigt.

Ergebnis: Eine Kopplung des Antibiotikums Ampicillin mit dem 2,5- Dihydroxybenzoesäuremethylester führt zu einer bisher unbekannten antimikrobiell wirksamen Verbindung. Die Ausbeute an Kopplungsprodukt beträgt 56%.

### Beispiel 13

### Herstellung neuer antimikrobieller Wirkstoffe durch Kopplung von 2,5-Dihydroxybenzoesäuren und -estern mit Sulfonamiden mittels Biotransformation

Methodik: Die Herstellung der Enzymlösung erfolgte nach Beispiel 1.

Der 2,5-Dihydroxybenzoesäuremethylester wird im äquimolaren Verhältnis mit p-Aminobenzensulfonamid (1 mM) in Natriumacetatpuffer (pH 5; 0,02 M) unter dem Einfluss einer Laccase (975 nmol/2 ml/min; gewonnen aus z.B. *Trametes versicolor* oder *Pycnoporus cinnabarinus*) 45 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographie gereinigt.

Ergebnis: Eine Kopplung des Antibiotikums p-Aminobenzensulfonamid mit dem 2,5-Dihydroxybenzoesäuremethylester führt zu einer bisher unbekannten antimikrobiell wirksamen Verbindung. Die Ausbeute an Kopplungsprodukt beträgt 66%.

### Beispiel 14

### Herstellung neuer antimikrobieller Wirkstoffe durch Kopplung von 2,5-Dihydroxybenzoesäuren und -estern mit substituierten Salicylsäuren mittels Blotransformation

Methodik: Die Herstellung der Enzymlösung erfolgte nach Beispiel 1.

Der 2,5-Dihydroxybenzoesäuremethylester wird im äquimolaren Verhältnis mit 4-Amino-2-hydroxybenzoesäure (1 mM) in Natriumacetatpuffer (pH 5; 0,02 M) unter dem Einfluss einer Laccase (975 nmol/2 ml/min; gewonnen aus z.B. *Trametes versicolor* oder *Pycnoporus cinnabarinus*) 45 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographie gereinigt.

Ergebnis: Eine Kopplung der 4-Amino-2-hydroxybenzoesäure mit dem 2,5-Dihydroxybenzoesäuremethylester führt zu einer bisher unbekannten antimikrobiell wirksamen Verbindung. Die Ausbeute an Kopplungsprodukt beträgt 63%.

### Beispiel 15

### Herstellung neuer antimikrobieller Wirkstoffe durch Kopplung von Hydrokaffeesäure mit 6-Aminopenicillansäure mittels Biotransformation

Methodik: Die,Herstellung der Enzymlösung erfolgte nach Beispiel 1.

Die 3(3,4-Dlhydroxyphenyl)propionsäure wird im äquimolaren Verhältnis mit 6-Aminopenlcillansäure (1 mM) In Natriumacetatpuffer (pH 5; 0,02 M) unter dem Einfluss einer Laccase (975 nmol/2 ml/min; gewonnen aus *Pycnoporus cinnabarinus*) 2 h bei Raumtemperatur umgesetzt. Zur Unterstützung der Umsatzgeschwindigkeit wird die Reaktionslösung bei 100 U/min geschüttelt.
Zur Aufarbeitung wird die Reaktionslösung mittels Oktadekanfestphase extrahiert. Die Elution des Produktes erfolgt mit Ethylacetat oder Acetonitril.

Ergebnis: Eine Kopplung des Antibiotikagrundkörpers 6-Amlnopeniclllansäure mit der Hydrokaffeesäure führt zu einer bisher unbekannten antimikrobiell wirksamen Verbindung mit hoher Aktivität.

### Beispiel 16

### Herstellung neuer antimikrobieller Wirkstoffe durch Kopplung von 2,5-Dihydroxy-N-(2-hydroxyethyl)benzamid mit Mercaptanen mittels Biotransformation

Methodik: Die Herstellung der Enzymlösung erfolgte nach Beispiel 1.

Das 2,5-Dihydroxy-N-(2-hydroxyethyl)benzamid wird im äquimolaren Verhältnis mit 3-Mercapto-1,2,4-triazol (1 mM) In Natriumacetatpuffer (pH 5; 0,02 M) unter dem Einfluß einer Laccase (975 nmol/2 ml/min; gewonnen aus z.B. *Trametes versicolor* oder *Pycnoporus cinnabarinus*) 50 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution des Produktes erfolgt mit Ethylacetat oder Acetonitril. Der nach der Entfernung des Lösungsmittels verbleibende Rückstand wird mit Hochleistungsflüssigchromatographie gereinigt.

Ergebnis: Eine Kopplung des 3-Mercapto-1,2,4-triazols mit dem 2,5-Dihydroxy-N-(2-hydroxyethyl)benzamids führt zu einer bisher unbekannten antimikrobiell wirksamen Verbindung. Die Ausbeute an Kopplungsprodukt beträgt 45%.

### Beispiel 17

### Herstellung neuer antimikrobieller Wirkstoffe durch Kopplung von 2,5-Dihydroxy-N-(2-hydroxyethyl)benzamid mit Mercaptanen mittels Blotransformation

Methodik: Die Herstellung der Enzymlösung erfolgte nach Beispiel 1.

Das 2,5-Dihydroxy-N-(2-hydroxyethyl)benzamld wird im äquimolaren Verhältnis mit 2-Mercapto-nicotinsäure (1 mM) In Natriumacetatpuffer (pH 5; 0,02 M) unter dem Einfluss einer Laccase (975 nmol/2 ml/min; gewonnen aus z.B.

*Trametes versicolor* oder *Pycnoporus cinnabarinus*) 50 min bei Raumtemperatur umgesetzt. Zur Unterstützung der Umsatzgeschwindigkeit wird die Reaktionslösung bei 100 U/min geschüttelt. Zur Aufarbeitung wird die Reaktionslösung mittels Oktadekanfestphase extrahiert. Die Elution der Produkte erfolgt mit Ethylacetat oder Acetonitril. Der nach der Entfernung des Lösungsmittels verbleibende Rückstand wird mit Hochleistungsflüssigchromatographle gereinigt.

Ergebnis: Eine Kopplung der 2-Mercapto-nicotinsäure mit dem 2,5-Dihydroxy-N-(2-hydroxyethyl)benzamlds führt zu bisher unbekannten antimikrobiell wirksamen Verbindungen. Die Ausbeute an Kopplungsprodukten beträgt 55%.

### Beispiel 18

### Prüfung der erfindungsgemäßen Kopplungsprodukte auf antimikrobielle Aktivität

Methodik: Es wurde der Agardiffusionstest nach Burkhardt (Burkhardt, F. (Hrsg.) Mikrobiologische Diagnostik. Georg-Thieme Verlag Stuttgart, New York 1992, S 724) durchgeführt. Die Prüfsubstanzen werden In abgestuften Konzentrationen auf Testplättchen Sensl-Disc (Becton Dickinson Microbiology Systems, Cockeysville, USA) aufgebracht. Für die Testung wurden Mueller-Hinton II-Agar In Stacker-Petrischalen (Becton Dickinson Microbiology Systems, Cockeysville, USA) eingesetzt. Als Teststamm wurden S. aureus Stamm ATCC 6538 ausgewählt. Die Einsaat dieses Teststamms wurde so gewählt, dass sich nach 16-20 h Bebrütung dicht stehende, jedoch nicht konfluierende Einzelkolonien entwickeln. Nach dem Trocknen der beimpften Nährböden werden maximal 6 Testplättchen mit leichtem Druck auf die Agaroberfläche aufgelegt. Nach 18 + 2 h Bebrütung bei 36°C werden die Hemmhöfe gemessen. Zur internen Qualitätssicherung wurde die Methode mit S. aureus ATCC 25923 überprüft.

Ergebnisse: Die durch die erfindungsgemäße Biotransformation erhältlichen Wirkstoffe sind antimikrobiell wirksam (Tab. 1). Die höchste antimikrobielle Wirksamkeit wird bei den Kopplungsprodukten nach Beispiel 4, 9 und 10 beobachtet.

**Tab. 1 Antibakterielle Wirksamkeit Im Agardiffusionstest nach Burkhardt gegen S. aureus ATCC 6538**

| Wirkstoff | Hemmhof (mm) | | | |
|---|---|---|---|---|
| | 250 µg | 125 µg | 50 µg | 10 µg |
| Kopplungsprodukt aus 6-Aminopenicillansäure und 2,5-Dihydroxy-N-(2-hydroxyethyl)benzamid gemäß Beispiel 1 | 24 | 22 | 18 | 12 |
| Kopplungsprodukt aus 7-Aminocephalosporinsäure und 2,5-Dihydroxy-N-(2-hydroxyethyl) benzamid Gemäß Beispiel 2 | 22 | 18 | 14 | r |
| Kopplungsprodukt aus 7-Aminodesacetoxycephalosporinsäure und 2,5-Dihydroxy-N-(2-hydroxyethyl)benzamid gemäß Beispiel 3 | 24 | 22 | 18 | r |
| Kopplungsprodukt aus Amoxicillin und 2,5-Dihydroxy-N-(2-hydroxyethyl)benzamid gemäß Beispiel 4 | >30 | >30 | >30 | >30 |
| Kopplungsprodukt aus p-Aminobenzensulfonamid und 2,5-Dlhydroxy-N-(2-hydroxyethyl)benzämid gemäß Beispiel 6 | 22 | 20 | r | r |
| Kopplungsprodukt aus 4-Amino-2-hydroxybenzoesäure und 2,5-Dihydroxy-N-(2-hydroxyethyl)benzamid gemäß Beispiel 7 | 10 | r | r | r |
| Kopplungsprodukt aus 6-Amlnopeniclllansäure und 2,5-Dihydroxybenzoesäuremethylester gemäß Beispiel 8 | | 30 | 24 | r |
| Kopplungsprodukt aus 7-Aminocephalosporinsäure und 2,5-Dlhydroxybenzoesäuremethylester gemäß Beispiel 9 | | 10 | r | r |
| Kopplungsprodukt aus 2,5-Dihydroxybenzoesäuremethylester und 7-Aminodesacetoxycephalosporinsäure gemäß Beispiel 10 | | 30 | 14 | r |
| Kopplungsprodukt aus Amoxicillin und 2,5-Dihydroxybenzoesäuremethylester gemäß Beispiel 11 | >30 | >30 | >30 | >30 |
| Kopplungsprodukt aus p-Aminobenzensulfonamid und 2,5-Dihydroxybenzoesäuremethylester gemäß Beispiel 13 | 10 | r | | |
| Kopplungsprodukt aus 4-Amino-2-hydroxybenzoesäure und 2,5-Dihydroxybenzoesäure-methylester gemäß Beispiel 14 | 10 | r | | |

### Beispiel 19

### Prüfung auf antimikrobielle Wirksamkeit an multireslstenten Keimen

Methodik: Die Prüfung erfolgte im Agardiffusiontest nach der im Beispiel 13 geschilderten Methodik. Als Testkeim wurde der multiresistente S. aureus Referenzstamm "Norddeutscher Stamm" (Smith Kline Beecham) eingesetzt.

Ergebnisse: Die Kopplungsprodukte sind überraschenderweise auch gegen multiresistente Keime teilweise hoch wirksam (Tab. 2). Die Wirksamkeit der Ausgangssubstanzen wird deutlich übertroffen (Tab. 4)

**Tab. 2 Antibakterielle Wirksamkeit im Agardiffusionstest nach Burkhardt gegen S. aureus "Norddeutscher Stamm"**

| Wirkstoff | Hemmhof mm | | | |
|---|---|---|---|---|
| | 250 µg | 125 µg | 50 µg | 10 µg |
| Kopplungsprodukt aus 6-Aminopenicillansäure und 2,5-Dihydroxy-N-(2-hydroxyethyl)-benzamid gemäß Beispiel 1 | 20 | 18 | 16 | r |
| Kopplungsprodukt aus 7-Aminocephalosporin-säure und 2,5-Dihydroxy-N-(2-hydroxyethyl)-benzamid Gemäß Beispiel 2 | 28 | 24 | 20 | r |
| Kopplungsprodukt aus 7-Amfnodesacetoxycephalosporinsäure und 2,5-Dihydroxy-N-(2-hydroxyethyl)benzamid gemäß Beispiel 3 | 24 | 22 | 20 | r |
| Kopplungsprodukt aus Amoxicillin und 10 2,5-Dihydroxy-N-(2-hydroxyethyl)benzamid gemäß Beispiel 4 | 24 | 22 | 18 | 10 |
| Kopplungsprodukt aus p-Amlnobenzensulfonamid und 2,5-Dihydroxy-N-(2-hydroxyethyl)benzamid gemäß Beispiel 6 | 26 | 22 | 18 | r |
| Kopplungsprodukt aus 4-Amino-2-hydroxybenzoesäure und 2,5-Dihydroxy-N-(2-hydroxyethyl)benzamid gemäß Beispiel 7 | r | r | r | r |
| Kopplungsprodukt aus 6-Aminopenicillansäure und 2,5-Dihydroxybenzoesäuremethylester gemäß Beispiel 8 | | 16 | r | r |
| Kopplungsprodukt aus 7-Aminocephalosporinsäure und 2,5-Dihydroxybenzoesäuremethylester gemäß Beispiel 9 | | r | r | r |
| Kopplungsprodukt aus 2,5-Dlhydroxybenzoe-säuremethylester und 7-Aminodesacetoxy-cephalosporinsäure gemäß Beispiel 10 | | r | r | r |
| Kopplungsprodukt aus Amoxicillin und 2,5-Dihydroxybenzoesäuremethylester gemäß Beispiel 11 | 20 | 16 | 10 | R |
| Kopplungsprodukt aus p-Aminobenzensulfon-amid und 2,5-Dihydroxybenzoesäuremethylester gemäß Beispiel 13 | 16 | 12 | | |
| Kopplungsprodukt aus 4-Amino-2-hydroxybenzoesäure und 2,5-Dihydroxybenzoesäuremethylester gemäß Beispiel 14 | r | r | | |

### Beispiel 20

Methodik: Die Prüfung erfolgte im Agardiffusiontest nach der im Beispiel 13 geschilderten Methodik. Als Testkeim wurde der aus Patientenmaterial isoliert multiresistente koagulasenegative *S. epidermidis* Stamm 99847 eingesetzt.

Ergebnisse : Auch gegen die schwer bekämpfbaren koagulasenegativen Staphylokokken, die z.B. bei der Besiedlung von Kathetermaterialien eine Rolle spielen und deshalb zu den nosokomialen Problemkeimen gehören, sind die erfindungsgemäßen Kopplungsprodukte wirksam. Die Wirksamkeit der Ausgangssubstanzen wird deutlich übertroffen (Tab. 4)

**Tab. 3 Antibakterielle Wirksamkeit im Agardiffusionstest nach Burkhardt gegen S. epidermidis**

| Wirkstoff | Hemmhof (mm) | | | |
|---|---|---|---|---|
| | 250 µg | 125 µg | 50 µg | 10 µg |
| Kopplungsprodukt aus 6-Aminopenicillansäure und 2,5-Dihydroxy-N-(2-hydroxyethyl)benzamid gemäß Beispiel 1 | r | r | r | r |
| Kopplungsprodukt aus 7-Aminocephalosporinsäure und 2,5-Dihydroxy-N-(2-hydroxyethyl)benzamid Gemäß Beispiel 2 | 22 | 22 | r | r |
| Kopplungsprodukt aus 7-Aminodesacetoxycephalosporinsäure und 2,5-Dihydroxy-N-(2-hydroxyethyl)benzamid gemäß Beispiel 3 | 20 | 24 | r | r |
| Kopplungsprodukt aus Amoxicillin und 2,5-Dihydroxy-N-(2-hydroxyethyl)benzamid gemäß Beispiel 4 | 30 | 28 | 18 | r |
| Kopplungsprodukt aus p-Aminobenzensulfonamid und 2,5-Dihydroxy-N-(2-hydroxyethyl)benzamid gemäß Beispiel 6 | 26 | 20 | r | r |
| Kopplungsprodukt aus 4-Amino-2-hydroxybenzoesäure und 2,5-Dihydroxy-N-(2-hydroxyethyl)benzamid gemäß Beispiel 7 | r | r | r | r |
| Kopplungsprodukt aus 6-Aminopenicillansäure und 2,5-Dihydroxybenzoesäuremethylester gemäß Beispiel 8 | | | | |
| Kopplungsprodukt aus 7-Aminocephalosporinsäure und 2,5-Dihydroxybenzoesäuremethyl-ester gemäß Beispiel 9 | | 14 | r | r |
| Kopplungsprodukt aus 2,5-Dihydroxybenzoesäuremethylester und 7-Aminodesacetoxycephalosporinsäure gemäß Beispiel 10 | | r | r | r |
| Kopplungsprodukt aus Amoxicillin und 2,5-Dihydroxybenzoesäuremethylester gemäß Beispiel 11 | 20 | 20 | 14 | r |
| Kopplungsprodukt aus p-Aminobenzensulfonamid und 2,5-Dihydroxybenzoesäuremethylester gemäß Beispiel 13 | 12 | r | | |
| Kopplungsprodukt aus 4-Amino-2-hydroxybenzoesäure und 2,5-Dihydroxybenzoesäuremethylester gemäß Beispiel 14 | r | r | | |

**Tab. 4 Antimikrobielle Wirksamkeit der Ausgangsstoffe**

| Wirkstoff | Konzentration/disc | Hemmhofgröße (mm) | |
|---|---|---|---|
| | | S. aureus "Nord.Stamm" | S. epidermidis |
| Ethlacetat | 0,25 | r | r |
| 2,5-Dihydroxybenzoesäure-methylester | 0,25 mg | r | r |
| 2,5-Dihydroxy-N-(2-hydroxyethyl)benzamid | 0,25 mg | 22 | 22 |
| | 0,05 mg | 14 | r |
| p-Aminobenzensolfonamid | 0,25 mg | r | r |
| 4-Amlno-2- hydroxybenzolsäure | 0,25 mg | r | r |
| Amoxicillin | 0,25 mg | 16 | 24 |
| | 0,12 mg | 14 | 20 |
| | 0,05 mg | 10 | 18 |
| | 0,01 mg | r | 12 |

### Beispiel 21

### Methode zur Stabilisierung der erfindungsgemäßen Kopplungsprodukte

Zur Erhöhung der Stabilität des Kreuzkopplungsproduktes eines äquimolaren Ansatzes aus Amoxicillin und 2,5-Dihydroxybenzoesäuremethylester, hergestellt entsprechend Beispiel 10, wurde eine spezielle Extraktionsmethode an Oktadekanfestphasen entwickelt. Hierzu wurde die Festphase mittels Methanol aktiviert und mit Aqua dest. equilibriert. Anschließend wurden x ml des wässrigen Kreuzkopplungsansatzes aufgetragen und nach dem Binden der Inhaltsstoffe für 20 min im Luftstrom getrocknet. Es schließt sich ein Waschschritt mit einem 10% Methanol/ 90% Aqua dest.- Gemisch an. Die Elution des Kreuzkopplungsproduktes erfolgte mit ebenfalls 100% entgastem Acetonitril. Der so gewonnene Extrakt wurde bei 4°C dunkel gelagert.

Ergebnis: Das Kopplungsprodukte ist innerhalb des Beobachtungszeitraums von 21 d stabil.

## Patentansprüche

1. Verfahren zur Herstellung von biologisch aktiven Wirkstoffen ausgehend von Arznei mitteln, **dadurch gekennzeichnet, dass** die Arznel-β-Lactam-Antibiotika sind,̅ und durch Einwirkung des radikalbildenden Enzyms Laccase EC 1.10.3.2.(Klassifikation gemäß Internationaler Enzym-Nomenklatur (Enzym Nomenclature, Academic Press, Inc, 1992, S. 24-154)) heteromolekular verknüpfte Kopplungsprodukte mit aromatischen Molekülen als Verbindungen mit modifiziertem Wirkungsspektrum erhältlich werden, die ein veränderte Verteilungsverhalten aufweisen mit der Maßgabe, dass das aromatische Molekül Benzol ist, das substituiert ist.

2. Verfahren nach Anspruch 1, wobei die Kopplungsprodukte in einer wässrigen Lösung mit einem pH-Wert von 2-8 bei Temperaturen zwischen 5°C und 60°C erhalten werden.

3. Verfahren nach Anspruch 1 und/oder 2 , wobei das aromatische Molekül optional mit einer oder mehreren funktionellen Gruppen oder Substituenten aus der Gruppe der Halogene; Sulfo-; Sulfon-; Sulfamino; Sulfanyl-; Amino- ; Amido-; Nitro-; Azo-; Imino-; Carboxy-; Cyano-; Formyl-; Halocarbonyl-; Carbamyl-; Carbamidoyl-; Phosphon-; Phosphonyl-; Cl-18 Alkyl-; Cl-18 -Alkenyl-; Cl-18 -Alkinyl-; Cl-18 -Alkoxy-; Cl-18 Oxycarbonyl-; Cl-18 -Oxoalkyl-; Cl-18 -Alkylsulfanyl-; Cl-18 -Alkylsulfonyl-; Cl-18 -Alkylimino- oder Alkylamino-Substituenten ausgestattet sind.

4. Verfahren gemäß mindestens einem der Ansprüche 1 bis 3, **dadurch gekennzeichnet, dass** die Kooplungsreaktion in Gegenwart mindestens eines Mediators aus der Gruppe der Hydroxylamine und/oder Hydroxamsäuren und ggf. eines Mediators aus der Gruppe der Amide stattfindet.

5. Verfahren gemäß Anspruch 1 bis 4, **dadurch gekennzeichnet, dass** die Reaktionsprodukte nach Beendigung der Reaktion stabilisiert werden.

## Claims

1. A method for producing biologically active agents starting from pharmaceutical preparations, **characterized in that** the pharmaceutical preparations are β lactam antibiotics and, by the action of the radical-forming enzyme laccase EC 1.10.3.2. (classification as per international enzyme nomenclature (Enzyme Nomenclature, Academic Press, Inc., 1992, p. 24 - 154)), heteromolecularly linked coupling products with aromatic molecules become available showing a modified spectrum of activity exhibiting a modified distribution behaviour, provided that the aromatic molecule is substituted benzene.

2. The method according to claim 1, wherein the coupling products are obtained in an aqueous solution having a pH value of 2 - 8 at temperatures of between 5°C and 60°C.

3. The method according to claim 1 and/or 2, wherein the aromatic molecule is optionally provided with one or more functional groups or substituents from the group consisting of halogens; sulfo substituents; sulfone substituents; sulfamino substituents; sulfanyl substituents; amino substituents; amido substituents; nitro substituents; azo substituents; imino substituents; carboxy substituents; cyano substituents; formyl substituents; halocarbonyl substituents; carbamyl substituents; carbamidoyl substituents; phosphone substituents; phosphonyl substituents; Cl-18 alkyl substituents; Cl-18 alkenyl substituents; Cl-18 alkinyl substituents; Cl-18 alkoxy substituents; Cl-18 oxycarbonyl substituents; Cl-18 oxoalkyl substituents; Cl-18 alkylsulfanyl substituents; Cl-18 alkylsulfonyl substituents; Cl-18 alkylimino substituents or alkylamino substituents.

4. The method according to at least one of the claims 1 to 3, **characterized in that** the coupling reaction takes place in the presence of at least one mediator from the group consisting of hydroxylamines and/or hydroxamic acids and, if necessary, a mediator from the group consisting of amides.

5. The method according to claims 1 to 4, **characterized in that** the reaction products are stabilized after completion of the reaction.

## Revendications

1. Procédé de fabrication de principes biologiquement actifs à partir de médicaments, **caractérisé en ce que** les médicaments sont des antibiotiques β-lactamiques et **en ce que**, par effet de l'enzyme formant des radicaux laccase EC 1.10.3.2. (classification selon la Nomenclature internationale des enzymes (Enzyme Nomenclature, Academic Press, Inc, 1992, pp. 24-154)), des produits de couplage à liaisons hétéromoléculaires contenant des molécules aromatiques peuvent être obtenus comme liaisons à spectre d'activité modifié présentant un comportement de répartition modifié, avec la consigne que la molécule aromatique qui est substituée est le benzène.

2. Procédé selon la revendication 1, où les produits de couplage sont obtenus dans une solution aqueuse ayant une valeur pH de 2 à 8 à des températures entre 5 °C et 60 °C.

3. Procédé selon la revendication 1 et/ou 2, où la molécule aromatique est pourvue facultativement d'un ou de plusieurs groupes fonctionnels ou substituants du groupe halogène, substituants sulfo, sulfon, sulfamino, sulfanyle, amino, amido, nitro, azo, imino, carboxy, cyano, formyle, halocarbonyle, carbamyle, carbamidoyle, phosphon, phosphonyle, alkyle Cl-18, alkényle Cl-18, alkinyle Cl-18, alkoxy Cl-18, oxycarbonyle Cl-18, oxoalkyle Cl-18, alkylsulfanyle Cl-18, alkylsulfonyle Cl-18, alkylimino Cl-18 ou alkylamino.

4. Procédé selon au moins l'une des revendications 1 à 3, **caractérisé en ce que** la réaction de couplage a lieu en présence d'au moins un médiateur du groupe des hydroxylamines et/ou des acides hydroxamiques et éventuellement d'un médiateur du groupe des amides.

5. Procédé selon la revendication 1 à 4, **caractérisé en ce que** les produits de réaction sont stabilisés une fois la réaction terminée.
